# EUROPEAN PATENT APPLICATION

(11) **EP 4 541 354 A1**
(43) Date of publication of application: **23.04.2025**
(21) Application number: 23383054.6
(22) Date of filing: 17.10.2023
(51) Int. Cl.: A61K 31/47, A61K 45/06, A61P 31/04

(54) **ELVITEGRAVIR TO TREAT MULTIDRUG-RESISTANT GRAM-POSITIVE BACTERIA INFECTIONS**

(71) Applicant: Fundación Privada Instituto De Salud Global Barcelona, 08036 Barcelona (ES); Fundació de Recerca Clínic Barcelona-Institut d'Investigacions Biomèdiques August Pi I Sunyer, 08036 Barcelona (ES); Universitat de Barcelona, 08028 Barcelona (ES); Fundació Privada Institut de Recerca de la SIDA-Caixa, 08916 Badalona (ES); Consorcio Centro de Investigación Biomédica en Red, 28029 Madrid (ES); Hospital Clínic de Barcelona, 08036 Barcelona (ES)
(72) Inventor: VILA ESTAPÉ, Jordi, 08036 BARCELONA (ES); BALLESTÉ DELPIERRE, Clara, 08036 BARCELONA (ES); MARTÍN VILARDELL, Núria, 08100 MOLLET DEL VALLÈS (ES); RUBIO, Elisa, 08036 BARCELONA (ES); MIRÓ MEDA, Josep Maria, 08036 BARCELONA (ES); PAREDES DEIRÓS, Roger, 08916 BADALONA (ES)
(74) Representative: ZBM Patents - Zea, Barlocci & Markvardsen

(57) **Abstract**

It relates to Elvitegravir or a pharmaceutically acceptable salt thereof for use in the treatment of a bacterial infection caused by multidrug-resistant Gram-positive bacteria.

## Description

The present invention relates to Elvitegravir's use as an antibacterial agent against different bacterial species.

### Background Art

Approved antiretroviral HIV (human immunodeficiency virus) drugs are divided into six drug classes based on how each drug interferes with the HIV life cycle. These six classes include the nucleoside reverse transcriptase inhibitors (NRTls), non-nucleoside reverse transcriptase inhibitors (NNRTls), protease inhibitors (Pls), entry inhibitors (divided into two types: fusion inhibitors and CCR5 antagonists), post-attachment inhibitors, and integrase strand transfer inhibitors (INSTls). Each class of drug attacks HIV in a different way. Generally, drugs from two (or sometimes three) classes are combined to ensure a powerful attack on HIV.

There are more than 37 million people living with HIV-1 (PLWH) in the world, but all human beings are at potential risk of acquiring HIV-1 at some point in their lifetime. Despite general progress in antiretroviral (ARV) treatment-based strategies to prevent and treat HIV-1 infection, according to the WHO (World Health Organisation) and the 2022 UNAIDS *Fact Sheet,* nearly 2 million new HIV-1 infections and more than 0.5 million deaths due to AIDS-related causes continue to occur every year.

Elvitegravir is an INSTI type of ARV drug used to treat HIV infections. Its formal name is 6-[(3-chloro-2-fluorophenyl)methyl]-1,4-dihydro-1-[(1S)-1-(hydroxymethyl)-2-methylpropyl]-7-methoxy-4-oxo-3-quinolinecarboxylic acid, with CAS number 697761-98-1 and having the following structure:

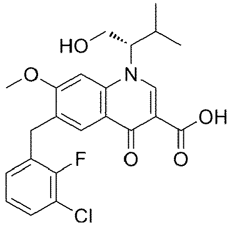

Elvitegravir for HIV treatment is only available in the combination tablets Genvoya^{®} and Stribild^{®}, both including another 3 drugs: Tenofovir (NRTI), Emtricitabine (NRTI) and Cobicistat
Antiretroviral treatment is effective in virtually all patients chronically infected with HIV-1, improving life expectancy and quality of life. However, people living with HIV under antiretroviral therapy are at higher risk of developing chronic complications and acquiring multidrug-resistant bacteria than healthy population. These factors have been associated with shifts in gut microbiota composition and immune activation. Several studies have observed differences in the composition of the gut microbiota between HIV-1 infected patients on ARV treatment and infected but untreated patients. However, as suggested by Li, S. X. et al. in "Complexities of Gut Microbiome Dysbiosis in the Context of HIV Infection and Antiretroviral Therapy", published in 2016 in Clin Pharmacol. Ther. 99, pp. 600-611, the gut microbiota of HIV patients on ARV treatment remains different from that of healthy patients, indicating that ARV treatment is not able to fully restore gut health after HIV infection. The authors explain that the strongest changes in gut microbiota in people suffering from HIV were associated to an increase in abundance of opportunistic pathogens, such as *Staphylococcus.*

PLWH are also at higher risk of MDR microorganisms' acquisition and infection, since they are more susceptible to present risk factors, such as frequent hospital admissions, higher rates of antibiotic intake and the previously mentioned chronic clinical complications and changes in intestinal microbiota composition. The emergence and dissemination of MDR organisms is an important public health problem due to high morbidity and mortality of infections caused by these microorganisms. Moreover, the development of new antibiotics has dropped considerably in recent years leaving limited effective treatment options against MDR bacteria. Some of these MDR bacterial strains, in particular *Staphylococcus aureus, Enterococcus faecium* and *Enterococcus faecalis,* are responsible for a great number of nosocomial infections that are difficult to treat and to eradicate.

Several antiretroviral drugs, such as nucleoside analogues Zidovudine (AZT) and Efavirenz (EFV), have shown *in vitro* antibacterial activity against gut and vaginal human commensal bacteria, suggesting that they could have a direct impact on gut microbiota. Ray, S. et al., in "Altered Gut Microbiome under Antiretroviral Therapy: Impact of Efavirenz and Zidovudine", published in 2021 in ACS Infect. Dis. 7, pp.1104-1115 discloses that AZT has activity against *E. coli, Bacteroides fragilis* and *Prevotella spp.,* whereas EFV has activity against *E. faecalis, Prevotella spp.* and *Bacteroides spp.* Additionally, Doléans-Jordheim, A. et al. in "Zidovudine (AZT) has a bactericidal effect on enterobacteria and induces genetic modifications in resistant strains", published in 2011 in the European Journal of Clinical Microbiology and Infectious Diseases 30, pp. 1249-1256 suggest the use of AZT in combination with known antibiotics, in particular gentamicin, against Gram-negative bacteria, some of which may be resistant. However, this document reports that AZT is not effective against Gram-positive bacteria, in particular *Staphylococcus aureus,* which is one of the most clinically relevant bacteria. Thus, although some studies suggest the potential use of ARVs as antibacterial agents, these are used in combination with "classical" antibiotics and they are not active against Gram-positive strains, let alone MDR resistant strains.

CN1 11 135182A discloses the combination of tannins and a quinolone drug, among which ELV is cited but not exemplified, as a possible antibacterial product. In the same line, Hena Yakoob in "Influence of the Anti-HIV drug Elvitegravir on Chlamydial Development and the Characterization of Chlamydial Polymorphic Membrane Protein Expression in Herpes Simplex Virus (HSV)/C. trachomatis Co-infected Cells", published in 2015 in an Undergraduate Honors Theses, discloses that low-dose quinolones show bacteriostatic activity on *Chlamydia trachomatis.* As mentioned before, people suffering from HIV are more exposed to bacterial infections due to their health condition, what could explain the fact that 1 in 20 people living with HIV/AIDS also suffer from chlamydial infections. However, although chlamydial infections can often be effectively treated with fluoroquinolones (such as levofloxacin), subinhibitory or low dose concentrations of these can produce fluoroquinolone-resistant mutants of *C*. *trachomatis.* Moreover, according to this document, ELV does not show bactericide effect against *Chlamydia* species, although it inhibits formation of infectious elementary bodies, which are the ones responsible of infecting host cells.

Finally, Zhang et al. in "HIV-1 integrase inhibitor-inspired antibacterials targeting isoprenoid biosynthesis", published in 2012 in ACS Medicinal Chemistry Letters 3, pp. 402-406 discloses an aryldiketo-acid that shows antibacterial activity against Gram-positive bacteria, such as *Bacillus anthracis, Listeria monocytogenes, Enterococcus faecium, Streptococcus pyogenes* and *S. aureus.* The article mentions that both monoketo and diketo-acids bind to similar domains present in prenyl transferases, inhibiting them in particular to UPPS, an essential enzyme in isoprenoid biosynthesis, which enables bacterial survival, for instance the development of bacterial cell wall, but monoketo an diketo-acids are divided into two different groups in this publication, having different activities.

Accordingly, from what is known in the art is derived that MDR bacteria is a growing public health problem and an effective antibacterial agent against these strains is needed. People suffering from HIV or hospitalized patients in general have a higher risk of being infected by these opportunistic pathogens and developing severe or chronic complications due to their immunosuppressed condition. Given that HIV-infected patients require combined treatments with several ARV drugs, they would highly benefit from a drug that is able to address the viral infection, while at the same time prevent and treat possible bacterial infections, reducing the number of drugs and thus possible drug-drug interactions, adverse effects, among other related disadvantages.

### Summary of Invention

The present inventors have found that Elvitegravir shows significant *in vitro* antibacterial activity against a set of clinically relevant multidrug-resistant Gram-positive bacteria, including Vancomycin-resistant enterococci (VRE) and Methicillin-resistant S. *aureus* (MRSA) strains, with Minimum Inhibitory Concentration (MIC) values of 4µg/mL when it is administered alone. Inventors have hence identified its potential use as a new antibacterial agent against multidrug-resistant Gram-positive bacteria.

The fact that ELV shows activity against bacteria which are resistant to known quinolone antibiotics, indicates that ELV's antibacterial effect may not solely depend on its quinolone-related structure. Moreover, although it is known that several molecules structurally related to INSTIs might inhibit bacterial growth by targeting isoprenoid biosynthesis in bacteria, the most potent inhibitors disclosed are aryldiketo acids. ELV is a quinolone derivative INSTI but it is a monoketo acid. As far as the inventors know, no antibacterial activity as such has been suggested in the prior art for this molecule or for any other pertaining to the same structural group.

The strong inhibitory activity observed against the above-mentioned strains, *S*. *aureus, E. faecium* and *E. faecalis,* including multidrug-resistant strains VRE and MRSA, is unexpected and unpredictable. As it is illustrated in the examples in a comparative way ELV has, little or no activity for Gram-negative strains and strong inhibitory activity against the above-mentioned Gram-positive strains, *S*. *aureus, E. faecium* and *E. faecalis,* including multidrug-resistant strains VRE and MRSA.

Accordingly, the present invention relates to Elvitegravir or a pharmaceutically acceptable salt thereof for use in the treatment of a bacterial infection caused by multidrug-resistant Gram-positive bacteria.

### Brief Description of Drawings

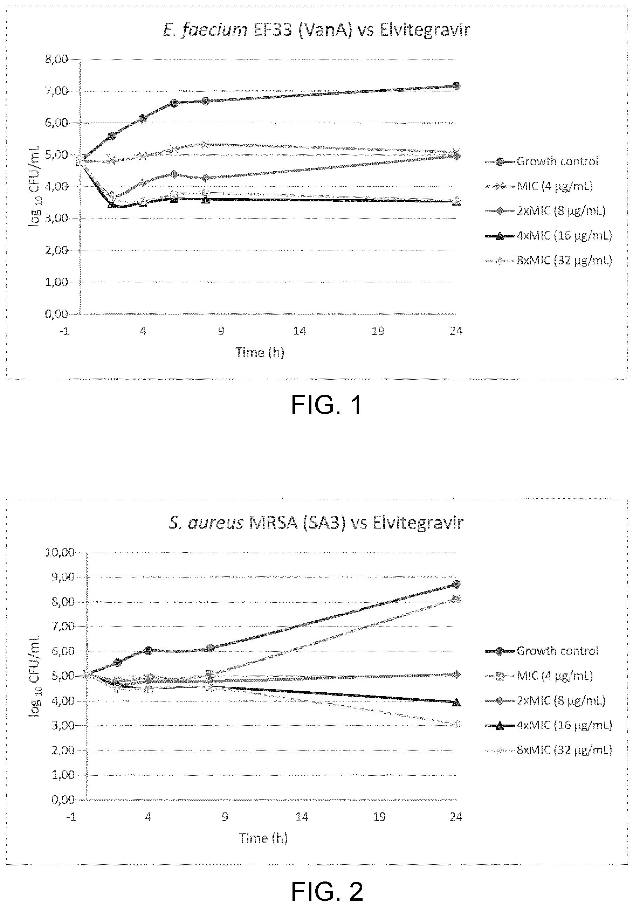

FIG. 1 shows a time-kill curve of *Enterococcus faecium* VanA vs. ELV, showing ELV's antibacterial activity with a MIC value of 4 µg/mL against VRE *Enterococcus,* resistant to both vancomycin and teicoplanin, according to an embodiment of the invention.
FIG. 2 shows a time-kill curve of *Staphylococcus aureus* MRSA vs. ELV, showing ELV's antibacterial activity with a MIC value of 4 µg/mL, according to an embodiment of the invention.

### Detailed description of the invention

### Definitions:

All terms as used herein in this application, unless otherwise stated, shall be understood in their ordinary meaning as known in the art. Other more specific definitions for certain terms as used in the present application are as set forth below and are intended to apply throughout the description and claims.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations. In this case, the dose may also vary depending on the intended effect of the drug, whether it is administered only as antibacterial agent, whether it is to prevent or to treat an infection caused by bacteria, or whether it is aimed to address both bacterial and viral infection at the same time.

The expression "pharmaceutically acceptable excipients or carriers" refers to pharmaceutically acceptable materials, compositions, or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The expression "methicillin-resistant *Staphylococcus aureus"* or the term "MRSA" refers to a multi-drug resistant strain of this bacteria, which is resistant to several beta-lactam antibiotics, such as methicillin. This strain may also be resistant to other types of antibiotics, such as quinolones.

The expression "vancomycin-resistant *Enterococcus"* or the term "VRE" refers to a multi-drug resistant strain of this bacteria, which is resistant to several glycopeptides, such as vancomycin. This strain may also be resistant to other types of antibiotics, such as beta-lactams and quinolones.

The term "patient" refers to a person who is designated to receive a medical treatment, therapy, or service. The term patient may be extended to an animal when used within the context of the animal receiving veterinary treatment or services.

The term "hospitalized" refers to a person who is receiving a medical treatment in hospital facilities, due to his/her medical condition or because the medical treatment may be only administered at a hospital. No temporary limitation is associated to this term, meaning that the patient may be short-term or long-term hospitalized.

The expression "pharmaceutically acceptable salt" refers to any salt formed, provided that it is a non-toxic salt, with an inorganic base selected from the group consisting of sodium hydroxide, potassium hydroxide, calcium hydroxide, magnesium hydroxide, ammonium hydroxide; or an or organic base selected from the group consisting of methylamine, diethylamine, triethylamine, triethanolamine, ethylenediamine, tris(hydroxymethyl)methylamine, guanidine, choline, cinchonine and the like; or an amino acid such as lysine, arginine, alanine, and the like.

As mentioned above, the present invention relates to Elvitegravir or its pharmaceutically acceptable salts for use in the treatment of a bacterial infection caused by multidrug-resistant Gram-positive bacteria. This aspect may also be formulated as the use of Elvitegravir or its pharmaceutically acceptable salts, as defined above, for the preparation of a medicament for the treatment of a bacterial infection caused by multidrug-resistant Gram-positive bacteria.

The present invention also relates to a method for the treatment of a bacterial infection caused by multidrug-resistant Gram-positive bacteria, comprising administering a therapeutically effective amount of Elvitegravir or its pharmaceutically acceptable salts, as defined above, together with pharmaceutically acceptable excipients or carriers, in a subject in need thereof, including a human.

In a particular embodiment, the Elvitegravir or its pharmaceutically acceptable salts for use as defined above, is that where the MDR Gram-positive bacteria are selected from the group consisting of *Staphylococcus aureus, Enterococcus faecium* and *Enterococcus faecalis.*

Inventors have hence identified ELV's potential concurrent use as an antiviral and antibacterial agent. In general terms, its use can enable to reduce the threat posed by multidrug-resistant Gram-positive bacteria, such as VRE and MRSA. Thus, in another particular embodiment, Elvitegravir or its pharmaceutically acceptable salts for use as defined above, is that where Elvitegravir or its salts also act simultaneously as antiviral agent and antibacterial agent, i.e., Elvitegravir or its pharmaceutically acceptable salts is for use in the treatment of a bacterial infection caused by multidrug-resistant Gram-positive bacteria and for use in the treatment of an antiviral infection.

As it is illustrated in the Examples, a total of 22 S. *aureus* clinical strains were tested by the present inventors, 10 of which were MRSA and 15 of which were resistant to three different quinolones. Some of them were both MRSA and quinolone-resistant, but, surprisingly, the 22 of them were susceptible to ELV, in general with a MIC value of 4 µg/mL (see Table 7 of Example 2). Thus, in a particular embodiment, Elvitegravir or a pharmaceutically acceptable salt thereof for use as defined above is that where the *Staphylococcus aureus* is multidrug-resistant *Staphylococcus aureus.* In another particular embodiment, Elvitegravir or its pharmaceutically acceptable salts for use as defined above, is that where the *Staphylococcus aureus* is methicillin-resistant *Staphylococcus aureus.* In another particular embodiment, Elvitegravir or its pharmaceutically acceptable salts for use as defined above, is that where the *Staphylococcus aureus* is resistant to one or more antibiotics selected from beta-lactams and quinolones.

Resistance to multiple antibiotics has increased rapidly in the past decades, especially among *E. faecium.* More than half of the pathogenic isolates of this species express resistance to one or more antibiotics, as can be seen from the results in the Examples of the present invention. In particular, the Examples also illustrate the results of a total of 22 *E. faecium* clinical strains tested, 11 of which were VRE, 8 were quinolone-resistant, 14 were resistant to beta-lactams and 5 of which were resistant to glycopeptides. Strains could be resistant to one or more of the mentioned antibiotics, hence being MDR strains. Surprisingly, all the strains were susceptible to ELV, with MIC values from 4 to 16 µg/mL (see Table 4 of Example 2). Accordingly, in a particular embodiment, Elvitegravir or its pharmaceutically acceptable salts for use as defined above, is that where the *Enterococcus* is a multi-drug resistant *Enterococcus.* In another particular embodiment, Elvitegravir or its pharmaceutically acceptable salts for use as defined above, is that where the *Enterococcus* is a vancomycin-resistant *Enterococcus.* In another particular embodiment, Elvitegravir or its pharmaceutically acceptable salts for use as defined above, is that where the *Enterococcus* is a multidrug-resistant *Enterococcus faecium.* In another particular embodiment, Elvitegravir or its pharmaceutically acceptable salts for use as defined above, is that where the *Enterococcus* is an *Enterococcus faecium* resistant to one or more antibiotics selected from glycopeptides, quinolones and beta-lactams.

Although a more reduced number of *E. faecalis* isolates show resistance to currently used antibiotics, it is predicted that this strain will also acquire resistance to other known antibiotics and thus ELV can play a major role in the treatment of infections caused by this bacterium. The examples also illustrate the results of a total of 16 *E*. *faecalis* clinical strains tested, where some of the strains were resistant to quinolones, in particular levofloxacin. Surprisingly, all strains were susceptible to ELV in MIC values from 4 to 16 µg/mL. Accordingly, in a particular embodiment, Elvitegravir or its pharmaceutically acceptable salts for use as defined above, is that where the *Enterococcus* is a multidrug-resistant *Enterococcus faecalis.* In another particular embodiment, Elvitegravir or its pharmaceutically acceptable salts for use as defined above, is that where the *Enterococcus* is an *Enterococcus faecalis* resistant to quinolones.

*Enterococci* are part of the normal intestinal microbiota, but *E. faecium* and E. *faecalis* species most commonly cause infections in humans, including endocarditis, urinary tract infections (UTls), intra-abdominal and pelvic infections, skin and soft tissue infections, and wound infection as well as concurrent bacteremia. Most common infections occur in the urinary tract, being the primary source for bacteremia caused by *Enterococci,* which is currently the second leading cause of healthcare-associated bacteremia. The major issue when dealing with enterococcal bacteremia is the presence of endocarditis, since it is most difficult to eradicate, currently requiring combined antibiotic treatment and increasing mortality rates from 26% to as high as 75%.

In addition, S. *aureus* is the leading cause of skin and soft tissue infections, but it can also cause serious infections such as bacteremia, pneumonia, osteomyelitis (bone infection), joint infections, septic arthritis, prosthetic device infections, meningitis, and toxic shock syndrome. MRSA infections have traditionally been associated to hospitalization or other healthcare-associated risk factors and HIV-infected population, and the latter, in particular, has significantly increased incidence of *S. aureus* bacteremia. Moreover, this pathogen is the most common cause of infective endocarditis (IE) and it is associated with an increased mortality, requiring prolonged i.v. (intravenous) antibiotics in combination therapy. ELV's use as antibacterial agent can also reduce MRSA infection rates when used as prophylaxis in patients who suffer from HIV-infection or belong to a group with risk factors, and especially reduce mortality rates and hospitalization time in patients already infected by MDR *S. aureus* that is difficult to eradicate.

Therefore, in a particular embodiment, Elvitegravir or its pharmaceutically acceptable salts for use as defined above, is that where the bacterial infection caused by MDR Gram-positive bacteria is selected from the group of skin infections, soft tissue infections, septic arthritis, prosthetic device infections, pulmonary infections, endocarditis, bacteremia, osteomyelitis, gastroenteritis, prostatitis, intra-abdominal and pelvic infections, meningitis, toxic shock syndrome, and a urinary tract infections. In another particular embodiment, Elvitegravir or its pharmaceutically acceptable salts for use as defined above, is that where the skin and soft tissue infections include impetigo, folliculitis, furuncles, carbuncles, abscesses, cellulitis, scalded skin syndrome. In another particular embodiment, Elvitegravir or its pharmaceutically acceptable salts for use as defined above, is that where the pulmonary infections include pneumonia and emphysema. In another particular embodiment, Elvitegravir or its pharmaceutically acceptable salts for use as defined above, is that where the urinary tract infections include upper urinary tract infections and lower urinary tract infections, such as cystitis, prostatitis, and epididymitis.

In a particular embodiment, Elvitegravir or its pharmaceutically acceptable salts for use as defined above, is that where the antibacterial agent is administered to a patient suffering from HIV. In this particular embodiment, Elvitegravir or its pharmaceutically acceptable salts is for use as defined above, where it may further comprise preventing a bacterial infection. In another particular embodiment, Elvitegravir as antibacterial agent is administered to a patient suffering from HIV, wherein the patient is a human being.

In a particular embodiment, Elvitegravir or its pharmaceutically acceptable salts for use as defined above, is that where the antibacterial agent is administered to a hospitalized patient. In another particular embodiment, Elvitegravir as antibacterial agent is administered to a hospitalized patient, wherein the patient is a human being.

In a particular embodiment, Elvitegravir or its pharmaceutically acceptable salts for use as defined above, is that where the Elvitegravir or its pharmaceutically acceptable salts is administered simultaneously, sequentially, or separately, together with one or more known antibacterial agents. In a more particular embodiment, the known antibacterial agents are selected from the group consisting of Amoxicillin, Cefazolin, Cefuroxime, Meropenem, Ertapenem, Imipenem, Ciprofloxacin, Levofloxacin, Vancomycin, Daptomycin, Metronidazole, Doxycycline, Clindamycin, Linezolid, Teicoplanin.

In a particular embodiment, Elvitegravir or its pharmaceutically acceptable salts for use as defined above, is that where the Elvitegravir or its pharmaceutically acceptable salt is administered simultaneously, sequentially, or separately, together with one or more antiviral agents. In a more particular embodiment, the additional antiviral agents are selected from the group consisting of Emtricitabine, Tenofovir, Lamivudine, Darunavir, Ritonavir, Lopinavir, Abacavir, Rilpivirine.

In a particular embodiment, Elvitegravir or its pharmaceutically acceptable salts for use as defined above, is that where Elvitegravir or a pharmaceutically acceptable salt thereof is for use in combination therapy with one or more known antibacterial agents and/or one or more antiviral agents.

In a particular embodiment, Elvitegravir or its pharmaceutically acceptable salts for use as defined above, is that where Elvitegravir or a pharmaceutically acceptable salt thereof is administered in the form of a pharmaceutical composition which comprises a therapeutically effective amount of Elvitegravir or a pharmaceutically acceptable salt thereof, together with pharmaceutically acceptable excipients or carriers. In another particular embodiment the therapeutically effective amount of Elvitegravir or its pharmaceutically acceptable salts for use as defined above ranges from 2 µg/mL to 16 µg/mL. In a more particular embodiment, the therapeutically effective amount of Elvitegravir or its pharmaceutically acceptable salts for use as defined above in ranges from 4 µg/mL to 8 µg/mL. In a more particular embodiment the therapeutically effective amount of Elvitegravir or its pharmaceutically acceptable salts for use as defined above against *Staphylococcus aureus* ranges from 2 µg/mL to 8 µg/mL. In a more particular embodiment, the therapeutically effective amount of Elvitegravir or its pharmaceutically acceptable salts for use as defined above against *Enterococcus faecium* and *Enterococcus faecalis* ranges from 2 µg/mL to 16 µg/mL.

In a particular embodiment, the pharmaceutically acceptable excipients or carriers may include diluents, extender agents, extender agents, disintegrants, stabilisers, preservatives, buffers, emulsifiers, flavouring agents, colouring agents, sweetening agents, thickeners, correctors, dissolution aids and other additives, generally known per se, such as water, vegetable oil, alcohol (e.g., ethanol or benzyl alcohol, etc.), polyethylene glycol, glyceryl triacetate, gelatine, carbohydrate (e.g. lactose, starch, etc.), magnesium stearate, talc, lanolin, petrolatum and the like.

In a particular embodiment, Elvitegravir or its pharmaceutically acceptable salts for use as defined above, is that where the composition is for oral administration or intravenous administration.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

Abbreviations: AMP: Amoxicillin, BIC: Bictegravir, CIP: Ciprofloxacin, ELV: Elvitegravir, LEV: levofloxacin, LZ: Linezolid, MOX: Moxifloxacin, MRSA: Methicillin-resistant *Staphylococcus* aureus, NIT: Nitrofurantoin, TEI: Teicoplanin, VAN: Vancomycin, VRE: Vancomycin-resistant enterococci, R: resistant, S: sensitive, I: intermediate.

### Methods

Four INSTI (integrase strand transfer inhibitor) class antiretroviral drugs, Bictegravir (BIC), Elvitegravir (ELV), Raltegravir potassium (RAL) and Dolutegravir (DTG) were tested by the inventors against several commensal bacteria to assess their antibiotic activity. Both BIC and ELV were kindly provided by Gilead Sciences Inc. (Foster City, California, USA), DTG was purchased from Quimigen (Madrid, Spain) and RAL was purchased from Merck Life Science (Madrid, Spain). Stock solutions of each ARV were prepared using dimethyl sulfoxide (DMSO), deionized water or methanol according to each ARV solubility (Table 1). Additionally, active antibiotics against the analyzed bacterial strains were purchased from Quimigen (Madrid, Spain).

Bacterial strains issued from the American Type Culture Collection (ATCC) were used for a first antibacterial activity screening of the tested antiretroviral drugs. For this screening, antibiotic susceptible strains were used, namely *Escherichia coli* ATCC 25922, *Klebsiella pneumoniae* ATCC 35657, *Acinetobacter baumannii* ATCC 17978, *Pseudomonas aeruginosa* ATCC 9027, *Enterococcus faecalis* ATCC 35657, and *Bacteroides thetaiotaomicron* ATCC 29741.

The antiretroviral drugs identified as having antibacterial activity in the first screening were further validated using a set of clinical strains showing different antibiotic resistance profiles. The selected bacterial strains were 22 *E. faecium,* 16 *E. faecalis, 22 Staphylococcus aureus* (45.45% (10/22) Methicillin-resistant *S. aureus* (MRSA), The antibiotic resistance profiles and sample origin of the selected clinical bacterial strains are shown on Supplementary Tables 3-7

The minimal inhibitory concentrations (MICs) of each ARV drug against the selected bacterial strains were determined by the broth microdilution method following Carpenter, D. E. et al. "Clinical and Laboratory Standards Institute (CLSI) Methods for Antimicrobial Susceptibility Testing of Anaerobic Bacteria", 9th Edition. CLSI M11 (2018). Briefly, anaerobic bacterial strains were inoculated in Blood Agar or Brucella agar for anaerobic bacteria and incubated at 37°C for 16-20h or for 44-48h under anaerobic atmosphere. Grown bacteria were resuspended in saline (0.9% sodium chloride) to obtain 0.5 McFarland turbidity standard, followed by a 100-fold dilution in Brucella broth supplemented with hemin (5 µg/mL), vitamin K1 (1 µg/mL), and lysed horse blood (5%) for anaerobic bacteria.

Stock solutions of each ARV were diluted in deionized water to a concentration of 1,024 µg/mL and further dilutions were performed using CAMHB or supplemented Brucella broth. The solvents methanol and DMSO were tested separately, and they did not affect bacterial growth at the assay conditions (≤5,12% v/v) (Table 1). Together, a final bacterial suspension of 7.5x105 CFU/mL was inoculated in a 96-well plate and the tested two-fold serial dilution range concentration for each ARV drug was 128-0.125 µg/mL. Wells containing no ARV and wells containing no bacteria were included as growth and negative control. Additionally, an active antibiotic against the bacterial strain was tested simultaneously as a positive control.

After incubation at 37°C for 44-48h under anaerobic conditions, MIC was determined visually as the lowest drug concentration that inhibited bacterial growth.

For the first antibacterial activity screening, all MICs were determined four times for each ARV-bacteria combination (two biological and two technical replicates), as *in-vitro* susceptibility testing. For the validation of ARV antibacterial activities using clinical strain, each MIC was determined once.

### Example 1: antibacterial susceptibility testing. Comparison of ELV with other INSTI class ARV drugs

Antibacterial activity of the ELV against a set of 5 bacterial strains representative of the human commensal microbiota and human pathogens is shown in Table 1A and a comparison of the activity against Gram-positive bacteria *E. faecalis* between ELV and three other drugs representative of INSTI class ARV drugs is shown in Table 1B.

**Table 1A. Minimum Inhibitory concentration (MIC) of ELV against a set of reference bacterial strains. Reported values are the range of four replicates (two biological replicates and two technical replicates). Bold indicates compounds with observed MIC at the given concentration. Solvent used for ELV used as positive control for each strain are indicated.**

| INSTI | Solvent | *E. faecalis* | *A. baumannii* | *P*. *aeruginosa* | *K. pneumoniae* | *B. thetaiotaomicron* |
|---|---|---|---|---|---|---|
| ELV | DMSO | 8-16 | >128 | >128 | >128 | >128 |

ELV only shows activity against the tested Gram-positive bacteria but not for the tested Gram-negative bacteria.

**Table 1B: Minimum Inhibitory concentration (MIC) of 4 INSTI class antiretrovirals against E. faecalis. Reported values are the range of four replicates (two biological replicates and two technical replicates). Solvents used for each antiretroviral are also indicated.**

| INSTI | Solvent | *E. faecalis* MIC (µg/mL) |
|---|---|---|
| ELV (example according to the invention) | DMSO | 8-16 |
| RAL (comparative example) | H₂O | >128 |
| DTG (comparative example) | DMSO | >128 |
| BIC (comparative example | DMSO | 64 |

As shown in the table, Elvitegravir (ELV) showed activity against *E. faecalis* with MIC values of 8-16 within the tested concentration range. BIC showed certain activity and RAL and DTG are not active.

### Example 2: antibacterial activity using clinical strains. Comparison of ELV and BIC.

The ARV antibacterial activity was tested in a set of clinical strains. *E. faecalis. E. faecium* and *S. aureus* clinical strains. A comparison with BIC was also conducted.

Table 2 shows MIC50 and MIC90 values of the tested ARVs against the several clinical strains. Remarkably, ELV showed antibacterial activity against *E. faecium* and *S. aureus* clinical strains with MIC values of 4-16 and 4 µg/mL respectively. The comparison with BIC showed that it has antibacterial activity against *E. faecium* clinical strains but with MIC values of 64 µg/mL.

**Table 2. Antibacterial activity of BIC and ELV against a set of clinical bacterial strains, ^{a}MIC₅₀ and ^{b}MIC₉₀ are defined as the minimal inhibitory concentration of drug capable of inhibiting 50% and 90% of the tested isolates, respectively.**

| Clinical strains | Number of tested strains | *^{a}*MIC₅₀ (µg/mL) | *^{b}*MIC₉₀ (µg/mL) |
|---|---|---|---|
| Bictegravir (BIC) Comparative example | | | |
| *E. faecium* | 22 | 64 | 64 |
| *E. faecalis* | 16 | 64 | 64 |

| Elvitegravir (ELV) (Example according to the invention) | | | |
|---|---|---|---|
| *E. faecium* | 22 | 4 | 16 |
| *E. faecalis* | 16 | 4 | 16 |
| *S. aureus* | 22 | 4 | 4 |

Additionally, individual MIC values for each ARV-clinical strain are shown in Tables 3-5. In all cases, the ARV antibacterial activity was not correlated to the clinical strain resistance mechanism or resistance profile to any of the tested antibiotics.

Tables 3-5. Individual MIC values for each ARV-clinical strain. Antibiotic resistance profiles, sample origin and tested antiretroviral minimal inhibitory concentrations of the selected clinical bacterial strains. Antibiotic susceptibility testing was performed by the disk diffusion method following EUCAST guidelines.

**Table 3. Enterococcus faecalis bacterial strains, 6 of which were resistant to quinolones.**

| Isolate number | AMP | VAN | TEI | LZ | LEV | NIT | Sample origin | BIC MIC (µg/mL) | ELV MIC (µg/mL) |
|---|---|---|---|---|---|---|---|---|---|
| EF1 | S | S | S | S | R | S | Urine | 64 | 8 |
| EF2 | S | S | S | S | S | S | Urine | 64 | 16 |
| EF3 | S | S | S | S | R | S | Urine | 64 | 16 |
| EF4 | S | S | S | S | S | S | Peritoneal fluid | 64 | 8 |
| EF5 | S | S | S | S | S | S | Wound swab | 64 | 4 |
| EF6 | S | S | S | S | S | - | Abscess | 64 | 8 |
| EF7 | S | S | S | S | S | - | Peritoneal fluid | 64 | 8 |
| EF8 | S | S | S | S | S | - | Urine | 64 | 8 |
| EF9 | S | S | S | S | S | S | Urine | 64 | 4 |
| EF10 | S | S | S | S | R | S | Urine | 64 | 4 |
| EF11 | S | S | S | S | R | S | Urine | 64 | 2 |
| EF12 | S | S | S | S | R | S | Urine | 64 | 4 |
| EF13 | S | S | S | S | S | S | Urine | 64 | 2 |
| EF14 | S | S | S | S | S | S | Urine | 64 | 8 |
| EF15 | S | S | S | S | R | S | Urine | 128 | 4 |
| EF16 | S | S | S | S | S | S | Urine | 128 | 8 |

**Table 4. Enterococcus faecium bacterial strains. 11 strains were resistant to vancomycin, 8 were quinolone-resistant, 14 were resistant to beta lactams and 5 were resistant to glycopeptides. Resistance mechanism of EFA12-EFA22 is VRE.**

| Isolate number | AMP | VAN | TEI | LZ | LEV | NIT | Sample origin | BIC MIC | ELV MIC |
|---|---|---|---|---|---|---|---|---|---|
| | | | | | | | | (µg/mL) | (µg/mL) |
| EFA1 | R | S | S | S | R | - | Wound swab | 64 | 16 |
| EFA2 | R | S | S | S | R | - | Abscess | 64 | 8 |
| EFA3 | R | S | S | S | R | - | Ascitic fluid | 32 | 8 |
| EFA4 | R | S | S | S | S | R | Urine | 64 | 16 |
| EFA5 | R | S | S | S | R | S | Urine | 128 | 16 |
| EFA6 | R | S | S | S | R | - | Intraabdominal collection | 64 | 2 |
| EFA7 | R | S | S | S | R | S | Urine | 64 | 4 |
| EFA8 | R | S | S | S | S | S | Urine | 64 | 8 |
| EFA9 | - | S | - | - | - | - | Rectal swab | 32 | 8 |
| EFA10 | - | S | - | - | - | - | Rectal swab | 64 | 8 |
| EFA11 | R | S | S | S | - | - | Wound swab | 64 | 16 |
| EFA12 | - | R | - | - | - | - | Rectal swab | 32 | 8 |
| EFA13 | R | R | R | S | R | - | Wound swab | 64 | 4 |
| EFA14 | - | R | - | - | - | - | Rectal swab | 32 | 4 |
| EFA15 | - | R | - | - | - | - | Rectal swab | 32 | 4 |
| EFA16 | - | R | - | - | - | - | Rectal swab | 32 | 4 |
| EFA17 | R | R | R | S | - | - | Bile | 64 | 4 |
| EFA18 | R | R | R | S | - | - | Ascitic fluid | 64 | 8 |
| EFA19 | R | R | S | S | - | - | Rectal swab | 32 | 2 |
| EFA20 | | R | S | S | - | - | Rectal swab | 32 | 4 |
| EFA21 | - | R | R | S | - | - | Rectal swab | 64 | 8 |
| EFA22 | R | R | R | S | R | R | Urine | 64 | 4 |

**Table 5. Staphylococcus aureus bacterial strains. 10 MRSA strains and 15 strains resistant to quinolones.**

| Isolate number | LEV | CIP | MOX | Sample origin | Resistance mechanism | ELV MIC (µg/mL) |
|---|---|---|---|---|---|---|
| SA1 | S | S | - | Blood | - | 4 |
| SA2 | S | S | - | Blood | - | 4 |
| SA3 | R | R | - | Abscess | MRSA | 4 |
| SA4 | S | R | - | Nasal swab | MRSA | 4 |
| SA5 | R | R | R | Blood | MRSA | 4 |
| SA6 | R | R | R | Facial wound | MRSA | 4 |
| SA7 | R | R | R | Muscle wound | | 4 |
| SA8 | R | R | R | Groin | MRSA | 2 |
| SA9 | R | R | R | Wound | - | 4 |
| SA10 | - | - | - | - | - | 4 |
| SA11 | S | I | S | - | - | 4 |
| SA12 | I | R | - | Arm pus | MRSA | 4 |
| SA13 | R | R | - | Wound | MRSA | 4 |
| SA14 | I | R | - | Abdominal wound | MRSA | 4 |
| SA15 | R | R | R | Wound | MRSA | 4 |
| SA16 | R | R | R | Wound | - | 4 |
| SA17 | R | R | R | Wound | MRSA | 4 |
| SA18 | R | R | R | Sputum | - | 8 |
| SA19 | R | R | R | - | - | 4 |
| SA20 | I | S | S | Wound | - | 4 |
| SA21 | S | S | S | Bronchial | - | 4 |
| SA22 | R | R | R | Wound | - | 4 |

### Results

The results showed that ELV has antibacterial activity against *Enterococcus spp.* and *Staphylococcus aureus* with MIC values of 4µg/mL, including VRE and MRSA strains.

### Citation List

### Patent Literature

- CN111135182A

### Non-Patent Literature

- Li, S. X. et al. in "Complexities of Gut Microbiome Dysbiosis in the Context of HIV Infection and Antiretroviral Therapy", Clin Pharmacol. Ther. 99, pp. 600-611 (2016).
- Ray, S. et al., in "Altered Gut Microbiome under Antiretroviral Therapy: Impact of Efavirenz and Zidovudine" ACS Infect. Dis. 7, pp.1104-1115 (2021).
- Doléans-Jordheim, A. et al. in "Zidovudine (AZT) has a bactericidal effect on enterobacteria and induces genetic modifications in resistant strains" European Journal of Clinical Microbiology and Infectious Diseases 30, pp. 1249-1256 (2011).
- Zhang et al. in "HIV-1 integrase inhibitor-inspired antibacterials targeting isoprenoid biosynthesis", ACS Medicinal Chemistry Letters 3, pp. 402-406 (2012).
- Hena Yakoob in "Influence of the Anti-HIV drug Elvitegravir on Chlamydial Development and the Characterization of Chlamydial Polymorphic Membrane Protein Expression in Herpes Simplex Virus (HSV)/C.trachomatis Co-infected Cells", Undergraduate Honors Theses, 2015.
- Carpenter, D. E. et al. "Clinical and Laboratory Standards Institute (CLSI) Methods for Antimicrobial Susceptibility Testing of Anaerobic Bacteria", 9th Edition. CLSI M11 (2018).

## Claims

1. Elvitegravir or a pharmaceutically acceptable salt thereof for use in the treatment of a bacterial infection caused by multidrug-resistant Gram-positive bacteria.

2. Elvitegravir or a pharmaceutically acceptable salt thereof for use according to claim 1, wherein the multidrug-resistant Gram-positive bacteria are selected from the group consisting of *Staphylococcus aureus, Enterococcus faecium* and *Enterococcus faecalis.*

3. Elvitegravir or a pharmaceutically acceptable salt thereof for use according to any of the claims 1-2, wherein the Elvitegravir or the salt thereof acts simultaneously as antiviral agent and antibacterial agent.

4. Elvitegravir or a pharmaceutically acceptable salt thereof for use according to any of the claims 1-3, wherein the multidrug-resistant Gram-positive bacteria is Methicillin-resistant *Staphylococcus aureus.*

5. Elvitegravir or a pharmaceutically acceptable salt thereof for use according to any of the claims 1-3, wherein the multidrug-resistant Gram-positive bacteria is a multidrug-resistant *Staphylococcus aureus* resistant to one or more antibiotics selected from beta-lactams, and quinolones.

6. Elvitegravir or a pharmaceutically acceptable salt thereof for use according to any of the claims 1-3, wherein the multidrug-resistant Gram-positive bacteria is Vancomycin-resistant *Enterococcus.*

7. Elvitegravir or a pharmaceutically acceptable salt thereof for use according to any of the claims 1-3, wherein the multidrug-resistant Gram-positive bacteria is an *Enterococcus faecium* resistant to one or more antibiotics selected from glycopeptides, quinolones, and beta-lactams.

8. Elvitegravir or a pharmaceutically acceptable salt thereof for use according to any of the claims 1-3, wherein the *Enterococcus* is an *Enterococcus faecalis* resistant to quinolones.

9. Elvitegravir or a pharmaceutically acceptable salt thereof for use according to any of the claims 1-8, wherein the bacterial infection caused by multidrug-resistant Gram-positive bacteria is selected from the group of a skin infection, a soft tissue infection, septic arthritis, a prosthetic device infection, a pulmonary infection, endocarditis, bacteremia, osteomyelitis, gastroenteritis, prostatitis, intra-abdominal and pelvic infection, meningitis, toxic shock syndrome, and a urinary tract infection.

10. Elvitegravir or a pharmaceutically acceptable salt thereof for use according to any of the claims 1-9, wherein the antibacterial agent is administered to a patient suffering from HIV.

11. Elvitegravir or a pharmaceutically acceptable salt thereof for use according to any of the claims 1-9, wherein the antibacterial agent is administered to a hospitalized patient.

12. Elvitegravir or a pharmaceutically acceptable salt thereof, for use according to any of the claims 1-11, wherein the Elvitegravir or the pharmaceutically acceptable salt thereof is administered simultaneously, sequentially or separately, together with one or more known antibacterial agents and/or one or more antiviral agents.

13. Elvitegravir or a pharmaceutically acceptable salt thereof, for use according to any of the claim 1-11, which is for use in combination therapy with one or more known antibacterial agents and/or antiviral agents.

14. Elvitegravir or a pharmaceutically acceptable salt thereof, for use according to any of the claims 1-13, wherein Elvitegravir or a pharmaceutically acceptable salt thereof is administered in the form of a pharmaceutical composition which comprises a therapeutically effective amount of Elvitegravir or a pharmaceutically acceptable salt thereof, together with pharmaceutically acceptable excipients or carriers.

15. Elvitegravir or a pharmaceutically acceptable salt thereof, for use according to any of the claims 1-14, wherein the composition is for oral administration or intravenous administration.
